# EUROPEAN PATENT APPLICATION

(11) **EP 4 603 600 A1**
(43) Date of publication of application: **20.08.2025**
(21) Application number: 23877770.0
(22) Date of filing: 13.10.2023
(51) Int. Cl.: C12Q 1/6886, C12Q 1/689, A61K 35/741, A61P 35/00

(54) **ORAL CANCER DIAGNOSIS BASED ON ORAL MICROBIOTA**

(30) Priority: 14.10.2022 KR 20220132140
(71) Applicant: National Cancer Center, Goyang-si, Gyeonggi-do 10408 (KR)
(72) Inventor: KIM, Mi Kyung, Goyang-si, Gyeonggi-do 10324 (KR); CHOI, Sung Weon, Seoul 06511 (KR)
(74) Representative: Algemeen Octrooi- en Merkenbureau B.V.
(86) International application number: PCT/KR2023/015885
(87) International publication number: WO 2024/080847

(57) **Abstract**

The present invention discloses the identification of microbial groups that significantly increase or decrease in the saliva of patients with oral cancer, compared to a control group, and relates to a composition for diagnosing oral cancer, comprising an agent for detecting the microbial groups, and a method for providing information for diagnosis of oral cancer using the microbes. The composition for diagnosing oral cancer and the method for providing information for diagnosis according to the present invention allow for the simple and accurate early diagnosis of the onset or potential development of oral cancer by analyzing and verifying microbial abundance levels that show significant changes compared to a normal control group.

## Description

### [Technical Field]

The present disclosure relates to a diagnosis of oral cancer based on oral microbiota, and more particularly, to a composition for diagnosing oral cancer based on oral microbiota, a kit for diagnosing oral cancer comprising the same, and a method for providing information for diagnosis of oral cancer.

### [Background Art]

The oral cavity, which is located at a gateway to a digestive system, harbors a complex community of bacteria that is considered the second largest microbial population in the human body after the gut. It is estimated that 700 species of bacteria that play an important role in maintaining oral health are present in the human oral cavity.

Recently, some research groups have reported the presence of oral microbes in other parts of the human body and the association between these microbes and numerous systemic diseases. In addition, recent studies have shown that the oral microbiota is strongly correlated with various cancer sites, such as colorectal cancer, esophageal cancer, and lung cancer. Among these studies, *Porphyromonas gingivalis* and *Fusobacterium nucleatum* are two oral species that have often shown strong carcinogenic potential, suggesting that the oral microbiome has value as a cancer biomarker.

Meanwhile, oral cancer is one of the most common malignant tumors worldwide, both in developed countries and especially in developing countries with high incidence and mortality rates. Oral squamous cell carcinoma (OSCC), the most common form (90% or more of oral cancer), is often diagnosed at a late stage, and has a poor quality of life and survival rate (< 50%) in most patients due to the complex management procedures of the primary tumor.

Therefore, continuous efforts are required for the early diagnosis of oral cancer and the development of biomarkers in order to improve the quality of life of these patients and reduce healthcare costs.

### [Disclosure]

### [Technical Problem]

The present disclosure aims to solve the aforementioned problems and other problems related thereto.

One exemplary object of the present disclosure is to provide a diagnostic composition capable of accurately and easily diagnosing oral cancer in a noninvasive manner, comprising an agent for detecting a species of microorganism that shows significant changes in abundance in oral cancer compared to a control group in a biological sample, particularly saliva.

Another exemplary object of the present disclosure is to provide a kit for diagnosing oral cancer, comprising a composition for diagnosing oral cancer.

Another exemplary object of the present disclosure is to provide a method for providing information for diagnosis of oral cancer, comprising: confirming the abundance of the microorganism from a biological sample, in particular saliva, and comparing the abundance of the microorganism in the biological sample with the abundance of the microorganism in a control sample.

The technical problems to be solved in accordance with the technical ideas of the present disclosure disclosed in the present specification are not limited to addressing those mentioned above, and other problems not mentioned will be clearly understood by those skilled in the art from the following description.

### [Technical Solution]

This will be specifically described as follows. Meanwhile, each of the descriptions and embodiments disclosed in the present application may also be applied to other descriptions and embodiments. That is, all combinations of various elements disclosed in the present application fall within the scope of the present application. In addition, the scope of the present application cannot be considered limited by the specific description set forth below.

An aspect of the present disclosure provides a composition for diagnosing oral cancer, comprising an agent for detecting microorganisms that show significant changes in abundance in patients with oral cancer compared to a control group.

As used herein, the term "oral cancer" refers to a general term for cancer occurring in the mouth (tongue, floor of the mouth, buccal mucosa, gums, palate, jawbone), lips, or oropharynx (the posterior part of the tongue where it connects to the throat), and specifically includes salivary gland cancer that occurs in the salivary gland, sarcoma that occurs in the jawbone or facial muscles, etc., malignant melanoma that forms black spots on the palate, gums, etc., and oral squamous cell carcinoma derived from the epithelial cells of the oral mucosa, among which about 90% of oral cancers are oral squamous cell carcinoma (OSCC), and the oral cancer of the present disclosure may be, but is not limited to, oral squamous cell carcinoma.

As used herein, the term "abundance" may refer to relative abundance or a relative presence ratio of a specific microbial species, and in particular, to the relative abundance based on presence ratio of specific microorganisms in the salivary microbiota that are associated with the diagnosis of oral cancer. However, it is not limited thereto.

As used herein, the "agent for detecting microorganisms" is not limited in the type of substance as long as it is an agent capable of detecting the presence of one or more selected from a group consisting of microorganisms that show a specific abundance in patients with oral cancer compared to a control group, for the prediction or diagnosis of oral cancer. For example, the agent for detecting microorganisms may be, but is not limited to, an antisense oligonucleotide, a primer pair, a probe, a peptide, a polynucleotide, an oligonucleotide, an aptamer, or an antibody that specifically binds to 16S rRNA of the microorganism.

Detection of microorganisms using the detection agent may be performed by an amplification reaction using one or more oligonucleotide primers that hybridize to a nucleic acid molecule encoding a microorganism-specific expression gene or a complement of the nucleic acid molecule, wherein the detection of the nucleic acid using the primers may be performed by amplifying a gene sequence using an amplification method such as PCR and then confirming whether the gene has been amplified by a method known in the art.

The "primer" refers to a polynucleotide or a variant thereof having a base sequence capable of complementarily bond to the terminus of a specific region of a gene used to amplify a specific region corresponding to a target site of a gene using PCR. The primer does not require to be completely complementary to the terminus of a specific region, and may be used as long as it is complementary enough to hybridize to the terminus to form a double-stranded structure.

The "probe" refers to a polynucleotide a variant thereof, or a polynucleotide and a labeling material attached thereto, having a base sequence capable of complementarily bond to a target site of a gen.

The "hybridization" refers to the pairing of two single-stranded nucleic acids with complementary base sequences to form a duplex structure, and hybridization can occur not only when the complementarity between the single-stranded nucleic acid sequences is a perfect match, but also when some mismatched bases are present.

The "antibody" (or immunoglobulin) refers to a substance that specifically binds to an antigen and causes an antigen-antibody reaction, and may be, for example, one or more selected from the group consisting of polyclonal antibodies, monoclonal antibodies, recombinant antibodies, or combinations thereof, and may specifically comprise polyclonal antibodies, monoclonal antibodies, recombinant antibodies, and a complete form having two full-length light chains and two full-length heavy chains, as well as functional fragments of antibody molecules, for example, Fab, F(ab'), F(ab')2, and Fv.

The "aptamer" is an oligonucleotide or peptide molecule, and the general description of the aptamer is described in detail in Bock LC et al., Nature 355(6360):5646(1992); Hoppe-Seyler F, Butz K "Peptide aptamers: powerful new tools for molecular medicine". J Mol Med. 78(8):42630(2000); Cohen BA, Colas P, Brent R. "An artificial cellcycle inhibitor isolated from a combinatorial library". Proc Natl Acad Sci USA. 95(24): 142727(1998) .

In addition, the diagnostic compositions of the present disclosure may further comprise a label that enables quantitative or qualitative measurement of the formation of an antigen-antibody complex, a conventional tool, a reagent, etc., used in immunological assays, in addition to an agent for measuring the presence or absence of the microorganisms.

As used herein, the term "diagnosis" refers to determining the susceptibility of a subject to a particular disease or condition, determining whether a subject currently has a particular disease or condition, determining the prognosis of a subject with a particular disease or condition (e.g., identifying a pre-metastatic or metastatic cancer state, determining the stage of cancer, or determining the cancer's response to treatment), or therametrics (e.g., monitoring the condition of a subject to provide information about treatment efficacy). For purposes of the present disclosure, the diagnosis is determining or predicting the onset or probability (risk) of developing oral cancer.

As used herein, "microorganisms that show significant changes in abundance in patients with oral cancer compared to a control group" refer to species of microorganisms that significantly increased or decreased in patients with oral cancer compared to a healthy control group. Specifically, it may be one or more microorganisms from 18 genera (*Abiotrophia*, *Actinomyces, Alloscardovia, Capnocytophaga, Dialister, Howardella, Leuconostoc, Mannheimia, Mogibacterium, Olsenella, Paraburkholderia, Parvimonas, Pasteurellaceae_uc, Porphyromonas, Prevotella, Saccharimonas, Sphingomonas,* and *Streptococcus*) and 16 species (AF385567_s, *Actinomyces odontolyticus, Alloprevotella tannerae, Capnocytophaga sputigena, Dialister pneumosintes, Fusobacterium periodonticum, Lactobacillus iners, Lactobacillus sakei, Leuconostoc gelidum, Neisseria elongate, Paraburkholderia graminis, Prevotella baroniae, Prevotella melaninogenica, Prevotella pallens, Streptococcus constellatus,* and *Streptococcus pneumoniae*), and in terms of high diagnostic accuracy (AUC values) and consistent results across all assays, it may be one or more microorganisms from 5 genera (*Actinomyces, Capnocytophaga, Porphyromonas, Prevotella,* and *Streptococcus*) and 4 species (*Fusobacterium periodonticum, Prevotella melaninogenica, Prevotella pallens,* and *Streptococcus pneumoniae*), but is not limited thereto.

In an embodiment of the present disclosure, microorganisms with significant differences in abundance between the oral microorganisms of a normal control group and patients with oral cancer have been identified, and it was confirmed that these microorganisms can be used as biomarkers for diagnosing oral cancer.

Based on the results of confirming the oral microbial population profiles of patients with oral cancer and a normal control group, in the present disclosure, at the phylum level, *Actinobacteria, Bacteroidetes, Firmicutes, Fusobacteria,* and *Proteobacteria* may be included as biomarkers for diagnosing oral cancer.

Specifically, the *Bacteroidetes, Fusobacteria,* and *Proteobacteria* may be characterized by a significant decrease in abundance in oral cancer compared to a normal control group, whereas *Actinobacteria* and *Firmicutes* may be characterized by a significant increase in abundance in oral cancer.

In the present disclosure, at the genus level, *Actinomyces, Streptococcus, Capnocytophaga, Granulicatella, Lautropia, Alloprevotella, Fusobacterium, Haemophilus, Porphyromonas, Prevotella,* and *Veillonella* may be included as biomarkers for diagnosing oral cancer.

Specifically, the *Actinomyces, Streptococcus, Capnocytophaga, Granulicatella,* and *Lautropia* may be characterized by a significant increase in abundance in oral cancer compared to a normal control group, whereas *Alloprevotella, Fusobacterium, Haemophilus, Porphyromonas, Prevotella,* and *Veillonella* may be characterized by a significant decrease in abundance in oral cancer compared to a normal control group.

In the present disclosure, at the species level of microorganisms, *Fusobacterium nucleatum, Fusobacterium periodonticum, Granulicatella adiacens, Haemophilus parainfluenzae,* KV831974_s, *Lautropia mirabilis, Neisseria meningitidis, Neisseria subflava,* PAC001345_s, *Porphyromonas pasteri, Prevotella histicola, Prevotella melaninogenica, Prevotella nanceiensis, Prevotella pallens, Prevotella salivae, Prevotella_uc, Streptococcus pneumoniae, Streptococcus salivarius, Streptococcus sanguinis, Veillonella dispar,* and *Veillonella parvula* may be included as biomarkers for diagnosing oral cancer.

Specifically, the *Fusobacterium nucleatum, Granulicatella adiacens, Lautropia mirabilis, Neisseria meningitidis, Streptococcus pneumoniae, and Streptococcus sanguinis* may be characterized by a significant increase in abundance in oral cancer compared to a normal control group, whereas *Fusobacterium peridonticum, Haemophilus parainfluenzae,* KV831974_s, *Neisseria subflava,* PAC001345_s, *Porphyromonas pasteri, Prevotella histicola, Prevotella melaninogenica, Prevotella nanceiensis, Prevotella pallens, Prevotella salivae, Prevotella_uc, Streptococcus salivarius, Veillonella dispar,* and *Veillonella parvula* may be characterized by a significant decrease in abundance in oral cancer compared to a normal control group.

As shown in the results of linear discriminant analysis of LEfSE for microbiota imbalance analysis, the fold change and AUC values of the relative abundance of these selected microorganisms support their use as biomarkers.

In addition, at the genus level, LASSO and RF analyses revealed that *Abiotrophia, Actinomyces, Alloscardovia, Capnocytophaga, Dialister, Howardella, Leuconostoc, Mannheimia, Mogibacterium, Olsenella, Paraburkholderia, Parvimonas, Pasteurellaceae_uc, Porphyromonas, Prevotella, Saccharimonas, Sphingomonas, and Streptococcus* may be included as biomarkers for diagnosing oral cancer. Here, the genus *Prevotella* and the genus *Streptococcus* may serve as particularly significant biomarkers, especially with an AUC value of 0.7 or more.

Specifically, the *Abiotrophia, Actinomyces, Capnocytophaga, Dialister, Mogibacterium, Parvimonas,* and *Streptococcus* may be characterized by an increase in abundance in oral cancer compared to a normal control group, whereas *Mannheimia, Pasteurellaceae_uc, Pophyromonas, Prevotella,* and *Saccharimonas* may be characterized by a significant decrease in abundance in oral cancer compared to a normal control group.

Furthermore, at the species level, AF385567_s, *Actionmyces odontolyticus, Alloprevotella tannerae, Capnocytophaga sputigena, Dialister pneumosintes, Fusobacterium periodonticum, Lactobacillus iners, Lactobacillus sakei, Leuconostoc gelidum, Neisseria elongate, Paraburkholderia graminis, Prevotella baroniae, Prevotella melaninogenica, Prevotella pallens, Streptococcus constellatus,* and *Streptococcus pneumoniae* may be included as biomarkers for diagnosing oral cancer.

Specifically, *Actionmyces odontolyticus, Alloprevotella tannerae, Capnocytophaga sputigena, Dialister pneumosintes, Neisseria elongate, Prevotella baroniae, Streptococcus constellatus,* and *Streptococcus pneumoniae* may be characterized by a decrease in abundance in oral cancer compared to a normal control group, whereas *Fusobacterium periodonticum, Prevotella melaninogenica,* and *Prevotella pallens* may be characterized by a significant decrease in abundance in oral cancer compared to a normal control group.

An exemplary embodiment of the present disclosure relates to a composition for diagnosing oral cancer, comprising an agent for detecting at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae*

In an exemplary embodiment of the present disclosure, the composition for diagnosing oral cancer may further comprise an agent for detecting at least one microorganism selected from the group consisting of the genus *Granulicatella,* the genus *Lautropia,* the genus *Alloprevotella,* the genus *Fusobacterium,* the genus *Haemophilus,* the genus *Veillonella, Fusobacterium nucleatum, Granulicatella adiacens, Haemophilus parainfluenzae, KV831974_s, Lautropia mirabilis, Neisseria meningitidis, Neisseria subflava, PAC001345_s, Porphyromonas pasteri, Prevotella histicola, Prevotella nanceiensis, Prevotella salivae, Prevotella_uc, Streptococcus salivarius, Streptococcus sanguinis, Veillonella dispar,* and *Veillonella parvula*

In an exemplary embodiment of the present disclosure, the composition for diagnosing oral cancer may further comprise an agent for detecting at least one microorganism selected from the group consisting of the genus *Dialister,* the genus *Mogibacterium,* the genus *Parvimonas,* the genus *Mannheimia,* the genus *Pasteurellaceae_uc,* the genus *Saccharimonas, Actinomyces odontolyticus, Alloprevotella tannerae, Capnocytophaga sputigena, Dialister pneumosintes, Neisseria elongate, Prevotella baroniae,* and *Streptococcus constellatus*

In an exemplary embodiment of the present disclosure, the composition for diagnosing oral cancer may further comprise an agent for detecting at least one microorganism selected from the group consisting of the genus *Howardella,* the genus *Paraburkholderia,* and the genus *Sphingomonas.*

Further, the agent for detecting at least one microorganism selected from the group consisting of the *genus Howardella,* the *genus Paraburkholderia,* the *genus Porphyromonas,* the *genus Capnocytophaga,* and the genus *Sphingomonas* may be for diagnosing early-stage oral cancer.

In an exemplary embodiment of the present disclosure, the composition for diagnosing oral cancer may further comprise an agent for detecting a gene encoding at least one of acyl-CoA dehydrogenase and dihydrofolate reductase, wherein the agent for detecting the gene may be for diagnosing early-stage oral cancer.

As used herein, the early-stage oral cancer may refer to stages 1 and 2 when divided into stages 1 to 4, depending on the size of the primary cancer, metastasis to lymph nodes in the neck, or distant metastasis. Specifically, stage 1 refers to a case where the primary cancer is 2 cm or less in size and has no metastasis to the lymph nodes in the neck or distant metastasis; stage 2 refers to a case where the primary cancer is 2 cm or more and 4 cm or less in size and has no metastasis to the lymph nodes in the neck or distant metastasis; stage 3 refers to a case where the primary cancer is 4 cm or more in size, has one lymph node metastasis of 3 cm or less in size in the neck, and has no distant metastasis; and stage 4 refers to a case where the primary cancer has invaded the bone or skin of the face, or the deep muscles of the tongue, or has one lymph node metastasis of 3 to 6 cm on the side of the primary site, a case where the primary cancer has two or more lymph node metastasis of 6 cm or less in the neck, or has metastasized to lymph nodes on both sides or on the opposite side of the lesion, with no distant metastasis, a case where the primary cancer has large lymph node metastasis of 6 cm or more in the neck, or a case where primary cancer is inoperable at the primary site, either without distant metastasis or with distant metastasis.

Another aspect of the present disclosure provides a kit for diagnosing oral cancer, comprising a composition for diagnosing oral cancer.

As used therein, the term "kit for diagnosing oral cancer" refers to a kit comprising a composition for diagnosing oral cancer, wherein the kit may further comprise, in addition to the composition for diagnosing oral cancer, instructions describing a method for using the kit for diagnosis.

As an example of the present disclosure, the kit may diagnose oral cancer if, compared to a normal human-derived sample, the abundance of *Actinomyces, Capnocytophaga, Granulicatella, Lautropia, Leptotrichia, Rothia,* and *Streptococcus* is increased, and the abundance of *Alloprevotella, Campylobacter, Fusobacterium, Haemophilus, Neisseria, Porphyromonas, Prevotella,* and *Veillonella is decreased* at the genus level, while if the abundance of *Fusobacterium nucleatum, Granulicatella adiacens, Lautropia mirabilis, Neisseria meningitides, Streptococcus pneumoniae,* and *Streptococcus sanguinis* is increased, and the abundance of *Fusobacterium periodonticum, Haemophilus parainfluenzae,* KV831974_s, *Neisseria subflava,* PAC001345_s, *Porphyromonas pasteri, Prevotella histicola, Prevotella melaninogenica, Prevotella nanceiensis, Prevotella pallens, Prevotella salivae, Prevotella_uc, Streptococcus salivarius, Veillonella dispar,* and *Veillonella parvula* is decreased at the species level.

Another aspect of the present disclosure provides a method for providing information for diagnosis of oral cancer.

The method for providing information comprises: (a) confirming the abundance of microbial species whose abundance is significantly altered in patients with oral cancer compared to a control group in a biological sample; and (b) comparing the abundance confirmed in step (a) with the abundance of microorganisms in a control sample.

As an example of the present disclosure, the "microbial species whose abundance is significantly altered in patients with oral cancer compared to a control group" may include one or more selected from the group consisting of *Actinomyces, Capnocytophaga, Granulicatella, Lautropia, Leptotrichia, Rothia, Streptococcus, Alloprevotella, Campylobacter, Fusobacterium, Haemophilus, Neisseria, Porphyromonas, Prevotella, and Veillonella* at the genus level, and *Fusobacterium nucleatum, Granulicatella adiacens, Lautropia mirabilis, Neisseria meningitides, Streptococcus pneumoniae, Streptococcus sanguinis, Fusobacterium periodonticum, Haemophilus parainfluenzae,* KV831974_s, *Neisseria subflava,* PAC001345_s, *Porphyromonas pasteri, Prevotella histicola, Prevotella melaninogenica, Prevotella nanceiensis, Prevotella pallens, Prevotella salivae, Prevotella_uc, Streptococcus salivarius, Veillonella dispar,* and *Veillonella parvula* at the species level.

As an example of the present disclosure, the "microbial species whose abundance is significantly altered in patients with oral cancer compared to a control group" may include one or more selected from the group consisting of the genus *Howardella,* the genus *Paraburkholderia,* and the genus *Sphingomonas.*

The step of confirming the abundance of a specific microbial species from the biological sample involves extracting DNA from the biological sample and analyzing the abundance of microorganisms.

As an example of the present disclosure, step (a) may involve analyzing the abundance of microorganisms in the oral cavity using an agent for detecting microorganisms according to an example of the present disclosure, for example, but is not limited to, extracting DNA from the microorganisms and using a set of PCR primers for 16S rRNA.

As an example of the present disclosure, step (b) involves comparing the results of the abundance analysis of step (a) with those of a healthy normal control group, wherein if the abundance of *Actinomyces, Capnocytophaga, Granulicatella, Lautropia, Leptotrichia, Rothia, Streptococcus, Howardella, Dialister, Paraburkholderia,* and *Sphingomonas* is increased, and the abundance of *Alloprevotella, Campylobacter, Fusobacterium, Haemophilus, Neisseria, Porphyromonas, Prevotella,* and *Veillonella* is decreased at the genus level, while if the abundance of *Fusobacterium nucleatum, Granulicatella adiacens, Lautropia mirabilis, Neisseria meningitides, Streptococcus pneumoniae,* and *Streptococcus sanguinis* is increased and the abundance of *Fusobacterium periodonticum, Haemophilus parainfluenzae,* KV831974_s, *Neisseria subflava,* PAC001345_s, *Porphyromonas pasteri, Prevotella histicola, Prevotella melaninogenica, Prevotella nanceiensis, Prevotella pallens, Prevotella salivae, Prevotella_uc, Streptococcus salivarius, Veillonella dispar,* and *Veillonella parvul* is decreased at the species level, it can be diagnosed as oral cancer or a risk of developing oral cancer.

As an example of the present disclosure, if the abundance of the genus *Howardella,* the genus *Paraburkholderia,* the genus *Capnocytophaga,* and the genus *Sphingomonas* is increased, or if the abundance of the genus *Porphyromonas* is decreased, it can be diagnosed as early-stage oral cancer.

The method for providing information may further comprise: confirming an expression level of a gene encoding at least one of an acyl-CoA dehydrogenase and a dihydrofolate reductase. If the expression level of the gene encoding the acyl-CoA dehydrogenase is increased, it can be diagnosed as early-stage oral cancer, while if the expression level of the gene encoding the dihydrofolate reductase is decreased, it can be diagnosed as early-stage oral cancer.

As used herein, the term "biological sample" refers to any substance, biological fluid, tissue or cell obtained from or derived from an individual, for example, whole blood, leukocytes, peripheral blood mononuclear cells, leukocyte buffy coat, plasma, serum, sputum, tears, mucus, nasal washes, nasal aspirate, breath, urine, semen, saliva, peritoneal washings, ascites, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, or cerebrospinal fluid, but preferably, a liquid biopsy for histopathological examination collected non-invasively from the subject to be diagnosed, for example, tissue, cells, blood, serum, plasma, saliva, sputum, or ascites, etc. from the subject to be diagnosed. As an example of the present disclosure, it may be saliva collected from the subject to be diagnosed.

Another aspect of the present disclosure provides a method for selecting a candidate drug for treating, ameliorating, or preventing oral cancer.

The method may comprise: (a) analyzing the abundance of at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae.* in a biological sample obtained from a subject before treatment with a candidate drug for treating, ameliorating, or preventing oral cancer;
(b) analyzing the abundance of at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae* in a biological sample obtained from a subject after treatment with the candidate drug; and
(c) screening the candidate drug by comparing the abundance of the microorganism analyzed in step (a) with the abundance of the microorganism analyzed in step (b).

Here, the analyzing of the abundance of the microorganisms in step (a) and step (b) is as described above in the method for providing information for diagnosing oral cancer.

In the present disclosure, the candidate drugs are not limited in type and may include, for example, any medicinal or functional food material, such as natural products, compounds, biomaterials, and prebiotics.

Another aspect of the present disclosure provides a method for predicting or monitoring oral cancer treatment response.

The method may comprise: (a) analyzing the abundance of at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae* in a biological sample obtained from a patient with oral cancer;
(b) analyzing the abundance of at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae* in a biological sample obtained from a patient after oral cancer treatment; and
(c) predicting, evaluating, or monitoring a treatment response by comparing the abundance of the microorganism analyzed in step (a) with the abundance of the microorganism analyzed in step (b).

Depending on the stage of oral cancer development and progression, it is important to suggest and evaluate appropriate therapies and establish treatment plans. By analyzing the abundance of microorganisms in a patient sample according to the present disclosure, it is possible to accurately predict and monitor the patient's treatment response.

Specifically, by analyzing and comparing the abundance of biomarker microorganisms in patient samples before and after treatment of a targeted oral cancer, it is possible to evaluate and monitor whether the oral cancer treatment results in a positive or negative treatment response, and to predict future treatment response.

Another aspect of the present disclosure provides a probiotic composition for treating, ameliorating, or preventing oral cancer.

In an embodiment of the present disclosure, for microorganisms whose abundance is reduced in a patent group with oral cancer compared to a control group, it is possible to treat, ameliorate, or prevent oral cancer by increasing the abundance in the oral cavity. Accordingly, the present disclosure provides a probiotic composition for treating, ameliorating, or preventing oral cancer, comprising at least one microorganism selected from the group consisting of the genus *Porphyromonas,* the genus *Prevotella, Fusobacterium periodonticum, Prevotella melaninogenica, Prevotella pallens,* the genus *Alloprevotella,* the genus *Fusobacterium,* the genus *Haemophilus,* the genus *Veillonella, Haemophilus parainfluenzae, KV831974_s, Lautropia mirabilis, Neisseria meningitidis, Neisseria subflava,* PAC001345_s, *Porphyromonas pasteri, Prevotella histicola, Prevotella nanceiensis, Prevotella salivae, Prevotella_uc, Streptococcus salivarius, Veillonella dispar, Veillonella parvula,* the genus *Mannheimia,* the genus *Pasteurellaceae_uc,* and the genus *Saccharimonas* whose abundance is reduced in a patient group with oral cancer compared to a healthy control group.

As used herein, the "probiotic" is defined as a live microorganism that provides a health benefit.

The probiotic compositions of the present disclosure may be provided in the form of a food or feed composition for human or animal consumption, or may be provided in the form of various over-the-counter drugs or pharmaceutical compositions for preventing, treating, or ameliorating oral cancer.

Another aspect of the present disclosure provides a method for diagnosing oral cancer.

Another aspect of the present disclosure provides a use of the composition for diagnosing oral cancer.

Another aspect of the present disclosure provides a method for treating, ameliorating, or preventing oral cancer.

Another aspect of the present disclosure provides a use of the probiotic composition for the manufacture of a medicament for treating, ameliorating, or preventing oral cancer.

### [Advantageous Effects]

The compositions for diagnosing oral cancer according to the present disclosure and the method for providing information for diagnosis of oral cancer using the same allow for the simple and accurate early diagnosis of the onset or probability of developing oral cancer by analyzing the abundance of biomarker microorganisms from biological samples and comparing the abundance analyzed in biological samples with the abundance of the microorganisms analyzed in a control group.

### [Description of Drawings]

FIG. 1 shows the results of confirming CHAO index (A), Shannon index (B), beta diversity (C, D), and the degree of overlap between groups at the species (E) and genus (F) levels in healthy control groups and patient groups with oral cancer, confirming that the bacterial diversity of the oral microbial population was significantly higher in patients with oral cancer.
FIG. 2 shows the oral microbial population showing the difference between healthy control groups and patient groups with oral cancer at the phylum (A), genus (B), and species (C) levels.
FIG. 3 shows the results of analyzing the functional imbalance and co-occurrence network of oral microorganisms.
FIG. 4 shows the results of confirming the POD and AUC values of selected genus-based markers in healthy control groups and patients with oral cancer, for the identification and validation of oral microbial genus-based markers of oral cancer.
FIG. 5 shows the results of confirming the POD and AUC values of selected species-based markers in healthy control groups and patients with oral cancer, for the identification and validation of oral microbial species-based markers of oral cancer.
FIG. 6 shows the results of confirming the difference in abundance of five oral microbiomes in control groups and patients with oral cancer at each stage of oral cancer, for the identification and validation of oral microbial genus-based markers of oral cancer.
FIG. 7 shows the results of confirming the AUC values of receiver operating characteristics (ROC) curves for five oral microbiomes, for the identification and validation of oral microbial genus-based markers of oral cancer
FIG. 8 shows the results of confirming the difference in abundance of two orthologs in control groups and patients with oral cancer at each stage of oral cancer.

### [Best Mode]

Hereinafter, the present disclosure will be described in more detail with reference to the following examples. However, these examples are intended to illustrate the present disclosure by way of example, and the scope of the present disclosure is not limited to these examples.

### Example 1. Sample preparation and sequencing

### 1-1. DNA extraction from study population sample

### (1) Study subjects and sample collection

The patient-control study enrolled 167 men and women (aged ≥ 19 years) diagnosed with histologically confirmed OSCC of the tongue, upper gums, lower gums, buccal mucosa, posterior palatine triangle, hard palate, soft palate, floor of the mouth, and lower lip at the National Cancer Centre (106 patients) and Seoul National University Dental Hospital (61 patients). 569 healthy controls without cancer at the time of enrollment were recruited from the National Cancer Center's cancer screening cohort, and subjects who had received treatment or surgery or used immunosuppressants were excluded at enrollment. All subjects signed a written informed consent form before enrollment and completed a detailed self-administered health and lifestyle questionnaire, including questions about alcohol consumption-related behaviors, at enrollment. This study was approved by the Institutional Review Board of the National Cancer Center (IRB No. NCC2018-0217). Saliva samples were collected in test tubes and immediately frozen at -80°C until further analysis.

### (2) DNA extraction and 16S rRNA gene amplicon sequencing

Bacterial DNA extraction was performed using the Fast DNA Spin extraction kit (MP Biomedical, Santa Ana, CA, USA) according to the manufacturer's instructions. Data quality was checked by uploading to the EzBioCloud 16S-based MTP app (ChunLab, Inc., Seoul, Korea), and DNA sequencing was subsequently performed using Illumina iSeq100 to generate single-end reads. The cloud app of Ezbiocloud software was used to detect and filter out low-quality sequences with read lengths (2,000 bp) and average Q values below 25. Non-redundant reads were denoised and extracted using DUDE-Seq software. The UCHIME algorithm was applied to the Ezbiocloud 16S chimera-free database to identify and remove chimeric sequences. The USEARCH program was used to perform taxonomic tasks to detect and calculate sequence similarity of query single-end reads against the EzBioCloud 16S database. EzBioCloud sequencing reads were clustered into OTUs with 97% sequence similarity using the UPARSE algorithm. The single-end reads from each sample were clustered into many OTUs using the UCLUST tool with the aforementioned cutoff values.

### 1.2. Study population characteristics

To investigate microbial population imbalances between groups, a total of 736 subjects (167 patients; 569 controls) were selected based on inclusion and exclusion criteria. Patients were further assigned to the discovery set or validation set based on the recruitment location, National Cancer Centre (106 patients) or Seoul National University Hospital (61 patients), while the control groups were randomly divided into 381 participants in the discovery set and 188 participants in the validation set. The demographic and clinical characteristics of patients with oral cancer and healthy control groups are summarized in Table 1.

**[Table 1]**

| Variables | | Discovery dataset | | | Validation dataset | | |
|---|---|---|---|---|---|---|---|
| | | Control group (N=381) | Patients (N=106) | P-value ^{a} | Control group (N=188) | Patients (N=61) | P-value^{a} |
| Sex | Male | 171 (44.9) | 65 (61.3) | <0.001 | 67 (35.6) | 34 (55.7) | <0.001 |
| | Female | 210 (55.1) | 41 (38.7) | | 121 (64.4) | 27 (44.3) | |
| Age (years) | Age (mean ± SD) | 55.8±8.0 | 63.2±13.9 | <0.001 | 61.0±8.1 | 68.0±11.7 | <0.001 |
| | I (age < 40) | 11 (2.9) | 6 (5.7) | <0.001 | 4 (2.1) | 2 (3.3) | <0.001 |
| | II (age < 50) | 35 (9.2) | 9 (8.5) | | 10 (5.3) | 3 (4.9) | |
| | III (age < 60) | 220 (57.7) | 30 (28.3) | | 48 (25.5) | 7 (11.5) | |
| | IV (age < 70) | 103 (27.0) | 22 (20.8) | | 112 (59.6) | 18 (29.5) | |
| | V (age ≥ 70) | 12 (3.1) | 39 (36.8) | | 14 (7.4) | 31 (50.8) | |
| Smoking^{b} | Current smoker | 51 (13.4) | 25 (23.6) | 0.07 | 13 (6.9) | 8 (13.1) | 0.001 |
| Drinking^{c} | Current drinker | 243 (63.8) | 39 (36.4) | <0.001 | 99 (52.7) | 20 (32.8) | 0.003 |
| T Stage | T0 | | 0 (0.0) | | | 2 (3.3) | <0.001 |
| | T1 | | 17 (16.0) | | | 14 (23.0) | |
| | T2 | | 18 (17.0) | | | 17 (27.9) | |
| | T3 | | 26 (24.5) | | | 1 (1.6) | |
| | T4 | | 35 (33.0) | | | 15 (24.6) | |
| | Undecid ed | | 10 (9.4) | | | 12 (19.7) | |
| N Stage | N0 | | 53 (50.0) | | | 34 (55.7) | 0.45 |
| | N1 | | 10 (9.4) | | | 6 (9.8) | |
| | N2 | | 14 (13.2) | | | 8 (13.1) | |
| | N3 | | 6 (5.7) | | | 0 (0.0) | |
| | Undecid ed | | 23 (21.7) | | | 13 (21.3) | |
| Stage | 0 | | 0 (0.0) | | | 1 (1.6) | 0.01 |
| | 1 | | 16 (15.1) | | | 12 (19.7) | |
| | 2 | | 13 (12.3) | | | 13 (21.3) | |
| | 3 | | 21 (19.8) | | | 4 (6.6) | |
| | 4 | | 45 (42.5) | | | 17 (27.9) | |
| | Undecid ed | | 11 (10.4) | | | 14 (23.0) | |
| Different iation | Poor | | 12 (11.3) | | | 0 (0.0) | <0.001 |
| | Moderat e | | 41 (38.7) | | | 4 (6.6) | |
| | Well | | 36 (34.0) | | | 40 (65.6) | |
| | Undecid ed | | 17 (16.0) | | | 17 (27.9) | |
| location | Lips | | 1 (0.9) | | | 0 (0.0) | 0.21 |
| | Tongue | | 30 (28.3) | | | 11 (18.0) | |
| | floor of the mouth | | 4 (3.8) | | | 1 (1.6) | |
| | Retromo lar trigone | | 5 (4.7) | | | 0 (0.0) | |
| | Hard plate | | 7 (6.6) | | | 5 (8.2) | |
| | Alveola r ridge d | | 45 (42.4) | | | 29 (47.6) | |
| | Others | | 14 (13.2) | | | 15 (24.6) | |

The results of the variables are presented as %. ^{a} P-value was calculated using the Kruskal-Wallis test for continuous variables and the chi-square test for categorical variables. ^{b} Smoking status was categorized into two groups (current smokers/current non-smokers). ^{c} Alcohol consumption status was also categorized into two groups (current drinkers/current non-drinkers). ^{d} Alveolar ridge: upper gums, lower gums, and buccal mucosa; ^{e} Others: maxillary sinus, soft palate, submandibular gland, and others. SD: standard error.

### Example 2. Confirmation of salivary microbial diversity in patients with oral cancer compared to healthy control groups

### 2.1. Analysis of sequencing results

### (1) Functional metagenomic analysis

For the EzBioCloud 16S-based MTP pipeline, the PICRUST algorithm was used to evaluate the functional profile of the microbial population identified by 16s rRNA sequencing. The raw sequencing reads were calculated using the EzBioCloud 16S microbiome Pipeline with default parameters and the identifiable reads aligned against the reference database. Functional abundance profiles of the oral microbial population were predicted (annotated) based on bioinformatics analysis, specifically using the Kyoto Encyclopedia of Genes and Genomes (KEGG) orthology, module, and pathway databases, by multiplying the number of gene vectors for each OUT by the abundance of the OUT in each sample. The predicted metagenomic profiles were classified into clusters of KEGG orthology, KEGG modules, and KEGG pathways, and compared between groups. The accuracy of each functional profile was determined by the nearest sequence taxon index.

### (2) Statistical analysis

Between-group differences in demographic and clinical characteristics of the subjects were compared using Kruskal-Wallis and chi-square tests for continuous and categorical variables, respectively. The alpha diversity of the CHAO and Shannon indices was calculated for each group, and the beta diversity was calculated by principal coordinate analysis (PCoA) based on weighted and unweighted UniFrac distances. Permutation multivariate analysis of variance (PERMANOVA) was performed to determine the significance of the between-group distance, and the diversity and PERMANOVA results were analyzed using the "GUniFrac" package in R. Differential abundance analyses were performed using Wilcoxon rank sum test and LEfSe method for genus, species level taxa and functional data KEGG pathways, KEGG modules, and KEGG orthologous taxa with default settings from the website https:/huttenhower.sph.harvard.edu/galaxy/root. To exclude small numbers of taxa, only genera and species with at least one sequence within at least 5% of all subjects were included. Heatmap correlation analysis was performed using the R package "heatmaply." Network models for correlations with |correlation coefficient| of 0.4 or more were displayed using Cytoscape version 3.9.0. The classification and functional composition profiles were subsequently analyzed based on fold-change analysis, multivariate-adjusted conditional logistic regression, and AUC values. To identify significant between-group differences, post hoc multiple comparisons using the Bonferroni method were used. Adjusted conditional logistic regression was performed to estimate ORs and corresponding 95% CIs adjusted for sex, age, smoking, and alcohol consumption. The AUC of receiver operating characteristics (ROC) curves was calculated using five-fold cross-validation in the discovery, validation, and full sets to characterize the diagnostic accuracy of the biomarkers for oral cancer and healthy control group conditions. All statistical tests were two-sided, with significance set at p < 0.05, and all other statistical analyses and visualizations were performed using the ggplot2 package in R version 4.1.1 (R Foundation for Statistical Computing, Vienna, Austria).

### 2.2. Confirmation of oral microbial population profiles in patients with oral cancer

### (1) Microbial population profiles showing differential abundance in patients with oral cancer

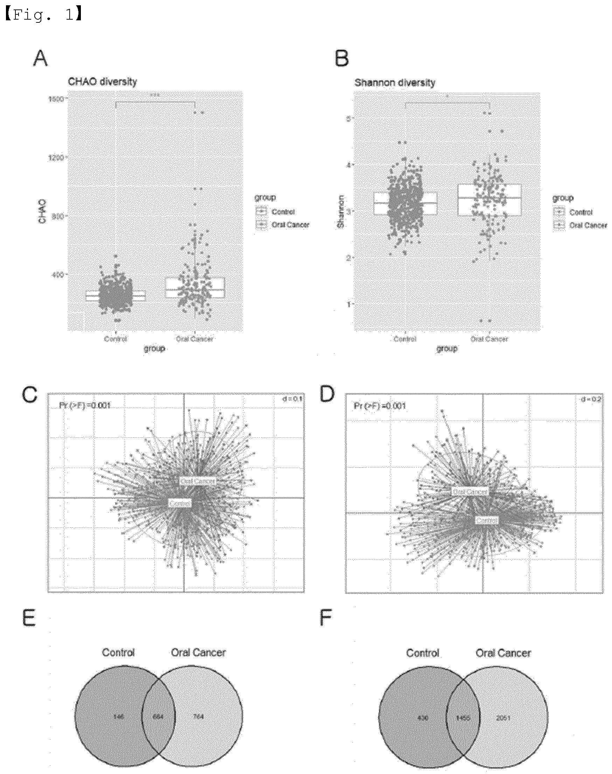

A total of 31 phyla, 116 classes, 244 orders, 492 families, 1574 genus, and 3936 species were identified in the study samples. In the analysis of alpha diversity, both CHAO and Shannon indices were significantly higher in patients with oral cancer compared to the control groups (FIGS. 1A and 1B). In addition, beta diversity, which represents the microbial population space between groups, was performed by Principal Coordinate Analysis (PCoA) based on weighted and unweighted UniFrac distances. The results of permutation analysis of the mutation test also showed that beta diversity was significantly different between patients with oral cancer and control groups (FIGS. 1C and 1D). Venn diagrams showed that 664 genera and 1,455 species overlapped between the two groups (FIGS. 1E and 1F).

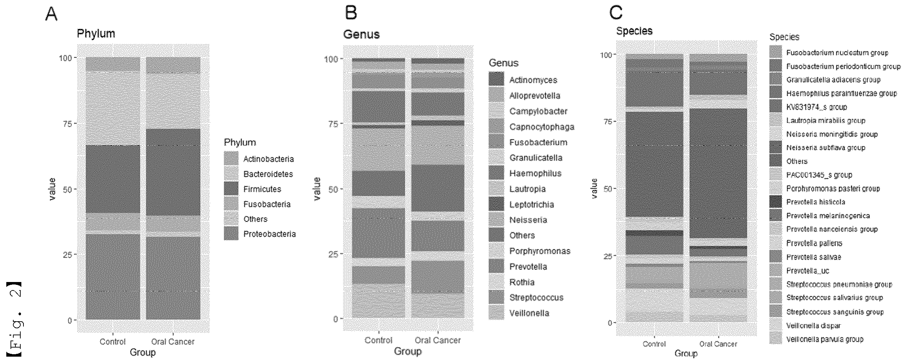

First, we analyzed the profiles of the top microbial population with a relative abundance of 1% or more, and at the phylum level, *Actinobacteria, Bacteroidetes, Firmicutes, Fusobacteria,* and *Proteobacteria* were the five most abundant phyla, accounting for 90% or more of the bacterial population (FIG. *2A*). *Bacteroidetes, Fusobacteria,* and *Proteobacteria* were significantly reduced in oral cancer, while *Actinobacteria* and *Firmicutes* were significantly enriched in oral cancer (FIG. 2A). At the genus level, the top 15 genera accounted for 86% or more of the salivary microbial population (FIG. 2B). *Actinomyces, Streptococcus, Capnocytophaga, Granulicatella,* and *Lautropia* were significantly higher in patients, whereas *Alloprevotella, Fusobacterium, Haemophilus, Porphyromonas, Prevotella, and Veillonella* were significantly lower in patients with cancer (FIG. 2B and Table 2). At the species level, all 21 species with relative abundance of 1% or more showed significant differences in oral cancer (Table 2). *Fusobacterium nucleatum, Granulicatella adiacens, Lautropia mirabilis, Neisseria meningitidis, Streptococcus pneumoniae,* and *Streptococcus sanguinis* were significantly higher in patients, while other species, including six Prevotella spp. were significantly reduced (FIG. 2C and Table 2).

**[Table 2]**

| Taxon level | Taxon name | Median abundance | | Fold change (FC) | | AUC^{b} |
|---|---|---|---|---|---|---|
| | | Patients | Control group | Patients/ Control group | p-value adjusted value^{a} | |
| Genus | *Actinomyces* | 1.18 | 0.82 | **1.43** | **1.54E-02** | 0.57 |
| | *Alloprevotella* | 0.86 | 2.07 | 0.42 | **2.62E-08** | 0.65 |
| | *Campylobacter* | 0.88 | 1.02 | 0.86 | 4.86E-02 | 0.55 |
| | *Capnocytophaga* | 1.26 | 0.73 | **1.72** | **1.32E-06** | 0.63 |
| | *Fusobacterium* | 3.45 | 4.53 | 0.76 | **2.26E-03** | 0.58 |
| | *Granulicatella* | 0.9 | 0.67 | **1.35** | 9. 65E-03 | 0.57 |
| | *Haemophilus* | 5.12 | 10.73 | 0.48 | **7.95E-11** | 0.67 |
| | *Lautropia* | 0.65 | 0.39 | **1.70** | **1.76E-02** | 0.56 |
| | *Leptotrichia* | 1.29 | 1.05 | **1.23** | 2.08E-01 | 0.53 |
| | *Neisseria* | 12.32 | 14.65 | 0.84 | 5.43E-02 | 0.55 |
| | *Porphyromonas* | 1.77 | 3.55 | 0.50 | **1.32E-06** | 0.62 |
| | *Prevotella* | 9.92 | 16.95 | 0.59 | **8.04E-14** | **0.70** |
| | *Rothia* | 2.11 | 1.87 | **1.13** | 7.44E-01 | 0.50 |
| | *Streptococcus* | 9.77 | 4.94 | **1.98** | **6.31E-17** | **0.72** |
| | *Veillonella* | 8.13 | 11.92 | 0.68 | **3.17E-08** | 0.65 |
| Species | *Fusobacterium nucleatum* group | 1.94 | 1.11 | **1.74** | **3.64E-05** | 0.61 |
| | *Fusobacterium periodonticum* group | 0.82 | 2.68 | 0.31 | **9.71E-22** | **0.75** |
| | *Granulicatella adiacens* group | 0.81 | 0.65 | **1.24** | **2.70E-02** | 0.55 |
| | *Haemophilus parainfluenzae* group | 4.88 | 10.17 | 0.48 | **8.14E-13** | 0.69 |
| | *KV831974_*s group | 0.77 | 1.01 | 0.76 | **6.36E-03** | 0.57 |
| | *Lautropia mirabilis* group | 0.65 | 0.39 | **1.70** | **1.33E-02** | 0.56 |
| | *Neisseria meningitidis* group | 0.99 | 0.25 | **4.01** | **1.86E-06** | 0.62 |
| | *Neisseria subflava* group | 6.59 | 12.14 | 0.54 | **1.44E-07** | 0.64 |
| | *PAC001345_s* group | 0.31 | 1.31 | 0.24 | **1.72E-17** | **0.72** |
| | *Porphyromonas pasteri* group | 0.5 | 2.05 | 0.25 | **8.14E-13** | 0.68 |
| | *Prevotella histicola* | 0.05 | 0.56 | 0.10 | **2.37E-10** | 0.67 |
| | *Prevotella melaninogenica* | 1.7 | 5.13 | 0.33 | **1. 38E-21** | **-0.75** |
| | *Prevotella nanceiensis* group | 0.16 | 0.99 | 0.16 | **2.95E-22** | **0.74** |
| | *Prevotella pallens* | 0.29 | 1.37 | 0.21 | **9.26E-15** | **0.70** |
| | *Prevotella salivae* | 0.4 | 0.69 | 0.59 | **5.83E-04** | 0.59 |
| | *Prevotella_uc* | 0.27 | 0.72 | 0.37 | **1.13E-10** | 0.67 |
| | *Streptococcus pneumoniae* group | 5.07 | 2.40 | **2.11** | **6.41E-17** | **0.72** |
| | *Streptococcus salivarius* group | 0.39 | 0.55 | 0.70 | **4.88E-04** | 0.40 |
| | *Streptococcus sanguinis* group | 2.39 | 1.24 | **1.93** | **1.20E-06** | 0.63 |
| | *Veillonella dispar* | 5.17 | 6.91 | 0.75 | **2.39E-03** | 0.57 |
| | *Veillonella parvula group* | 1.53 | 2.50 | 0.61 | **2.35E-05** | 0.61 |

^{a} P-value was calculated by Kruskal-Wallis using a false discovery rate (FDR) developed by Benjamini-Hochberg. ^{b} The area under the curve (AUC) of receiver operating characteristics (ROC) curve was calculated using five-fold cross-validation on the entire data set to characterize the general diagnostic accuracy of patients with oral cancer versus healthy control groups.

To investigate the oral microbiota imbalance in patients with cancer compared to healthy control groups, a linear discriminant analysis of effect size (LEfSe) was performed at the genus and species level, and only genera and species with one or more sequences in at least 5% of all subjects were included to exclude small numbers of taxa.

Based on the results of LEfSe, 19 genera and 39 species were identified that were not significantly modulated between the groups. As shown in Table 2, with the exception of one non-significant genus in the top dominant microbiome, 11 of the 15 genera and all 21 species were repeatedly identified as significant biomarkers in the LEfSe results.

To further investigate the association between oral cancer risk and the genera and species with high abundance in patients, multiple conditional logistic regression was performed, adjusted for sex, age, smoking, and drinking status (Table 3, Associations between oral cancer risk and the top 15 genera and 21 species in the entire study population). Each taxon was tested as a continuous variable and as a categorical variable defined by tertile distribution among healthy control groups. At the genus level, *Actinomyces, Capnocytophaga, Lautropia,* and *Streptococcus* were significantly associated with an increased risk of oral carcinogenesis, with higher risks identified in the highest tertile compared to the lowest tertile. In contrast, *Fusobacterium, Haemophilus, Porphyromonas, Prevotella,* and *Veillonella* showed significant negative associations with higher risks in the lowest tertile compared to the highest tertile.

**[Table 3]**

| Taxon level | Taxon name | Patient s/ Control group | Tertiles ^{a} | Contro l group (n=569 ) | Oral cancer (n=167) | Multivari able OR (95% CI)^{b} | P-value adjusted value ^{c} |
|---|---|---|---|---|---|---|---|
| | *Actinomyces* | up | 3 (>1.19) | 190 | 83 | 1.76 (1.1-2.85) | **1. 88E-02** |
| | | | 2 (0.57-1.19) | 189 | 36 | 0.76 (0.44-1.31) | 3.25E-01 |
| | | | 1 (<0.57) | 190 | 48 | ref | |
| | | | Continuo us scale | | | 1.22 (1.09-1.37) | **8.57E-04** |
| | *Capnocytoph aga* | up | 3 (>1.15) | 190 | 89 | 2.58 (1.58-4.31) | **2.09E-04** |
| | | | 2 (0.47-1.15) | 189 | 41 | 1.1 (0.62-1.93) | 7.51E-01 |
| | | | 1 (<0.47) | 190 | 37 | ref | |
| **Genus** | | | Continuo us scale | | | 1.36 (1.2-1.57) | **4.24E-06** |
| | *Granulicate lla* | up | 3 (>0.98) | 190 | 77 | 1.29 (0.8-2.1) | 2.91E-01 |
| | | | 2 (0.47-0.98) | 189 | 44 | 0.87 (0.52-1.46) | 5.99E-01 |
| | | | 1 (<0.47) | 190 | 46 | ref | |
| | | | Continuo us scale | | | 1.05 (0.95-1.16) | 3.13E-01 |
| | *Lautropia* | up | 3 (>0.70) | 190 | 81 | 1.79 (1.13-2.87) | **1. 44E-02** |
| | | | 2 (0.18-0.70) | 189 | 33 | 0.61 (0.35-1.06) | 8.17E-02 |
| | | | 1 (<0.18) | 190 | 53 | ref | |
| | | | Continuo us scale | | | 1.14 (1.06-1.24) | **6.29E-04** |
| | *Leptotrichi a* | up | 3 (>1.48) | 190 | 74 | 1.17 (0.73-1.88) | 5.03E-01 |
| | | | 2 (0.73-1.48) | 189 | 37 | 0.6 (0.35-1.02) | 6.04E-02 |
| | | | 1 (<0.73) | 190 | 56 | ref | |
| | | | Continuo us scale | | | 1.07 (0.96-1.18) | 2.15E-01 |
| | *Rothia* | up | 3 (>2.88) | 190 | 64 | 0.8 (0.49-1.28) | 3.48E-01 |
| | | | 2 (1.17-2.88) | 189 | 44 | 0.67 (0.41-1.11) | 1.21E-01 |
| | | | 1 (<1.167) | 190 | 59 | ref | |
| | | | Continuo us scale | | | 0.99 (0.94-1.04) | 5.98E-01 |
| | *Streptococc us* | up | 3 (>6.47) | 190 | 118 | 3.83 (2.27-6.67) | **9.67E-07** |
| | | | 2 (3.81-6.47) | 189 | 25 | 1.01 (0.53-1.93) | 9.78E-01 |
| | | | 1 (<3.81) | 190 | 24 | ref | |
| | | | Continuo us scale | | | 1.07(1.04 -1.09) | **1. 90E-07** |
| | *Alloprevote lla* | down | 3 (>3.16) | 190 | 36 | ref | |
| | | | 2 (1.23-3.16) | 189 | 28 | 0.76 (0.42-1.36) | 3.53E-01 |
| | | | 1 (<1.23) | 190 | 103 | 2.39 (1.48-3.93) | **4.75E-04** |
| | | | Continuo us scale | | | 0.96 (0.9-1.03) | 3.01E-01 |
| | *Campylobact er* | down | 3 (>1.39) | 190 | 52 | ref | |
| | | | 2 (0.68-1.39) | 189 | 45 | 0.9 (0.54-1.51) | 7.03E-01 |
| | | | 1 (<0.68) | 190 | 70 | 1.33 (0.83-2.15) | 2.39E-01 |
| | | | Continuo us scale | | | 0.86 (0.69-1.06) | 1.65E-01 |
| | *Fusobacteri* um | down | 3 (>5.84) | 190 | 43 | ref | |
| | | | 2 (3.30-5.84) | 189 | 47 | 1.27 (0.75-2.18) | 3.76E-01 |
| | | | 1 (<3.30) | 190 | 77 | 2.26 (1.38-3.77) | **1. 51E-03** |
| | | | Continuo us scale | | | 0.92 (0.86-0.97) | **6.88E-03** |
| | *Haemophilus* | down | 3 (>13.61) | 190 | 38 | ref | |
| | | | 2 (8.16-13.61) | 189 | 26 | 0.51 (0.28-0.94) | **3.20E-02** |
| | | | 1 (<8.16) | 190 | 103 | 2.09 (1.29-3.42) | **2.96E-03** |
| | | | Continuo us scale | | | 0.96 (0.93-0.98) | **1. 74E-03** |
| | *Neisseria* | down | 3 (>20.81) | 190 | 55 | ref | |
| | | | 2 (9.31-20.81) | 189 | 43 | 0.76 (0.45-1.28) | 3.06E-01 |
| | | | 1 (<9.31) | 190 | 69 | 1.17 (0.73-1.88) | 5.25E-01 |
| | | | Continuo us scale | | | 1 (0.98-1.01) | 6.98E-01 |
| | *Porphyromon as* | down | 3 (>5.73) | 190 | 31 | ref | |
| | | | 2 (2.04-5.73) | 189 | 43 | 1.58 (0.89-2.84) | 1.20E-01 |
| | | | 1 (<2.04) | 190 | 93 | 3.02 (1.81-5.18) | **3.59E-05** |
| | | | Continuo us scale | | | 0.93 (0.88-0.98) | **5.77E-03** |
| | *Prevotella* | down | 3 (>23.25) | 190 | 22 | ref | |
| | | | 2 (12.58-23.25) | 189 | 41 | 1.78 (0.97-3.34) | 6.47E-02 |
| | | | 1 (<12.58) | 190 | 104 | 4.11 (2.38-7.34) | **7.73E-07** |
| | | | Continuo us scale | | | 0.94 (0.92-0.96) | **8.57E-09** |
| | *Veillonella* | down | 3 (>15.82) | 190 | 25 | ref | |
| | | | 2 (8.54-15.82) | 189 | 52 | 2.71 (1.53-4.94) | **8. 61E-04** |
| | | | 1 (<8.54) | 190 | 90 | 3.58 (2.07-6.38) | **8.08E-06** |
| | | | Continuo us scale | | | 0.94 (0.92-0.97) | **6.47E-05** |
| | *Fusobacteri* um *nucleatum* group | up | 3 (>1.84) | 190 | 86 | 2 (1.23-3.3) | **5. 73E-03** |
| | | | 2 (0.65-1.84) | 189 | 43 | 0.98 (0.57-1.69) | 9.47E-01 |
| | | | 1 (<0.65) | 190 | 38 | ref | |
| | | | Continuo us scale | | | 1.15 (1.06-1.24) | **5.70E-04** |
| | *Granulicate lla adiacens* group | up | 3 (>0.96) | 190 | 71 | 1.13 (0.7-1.85) | 6.13E-01 |
| | | | 2 (0.45-0.96) | 189 | 50 | 1.1 (0.66-1.82) | 7. 14E-01 |
| **Species** | | | 1 (<0.45) | 190 | 46 | ref | |
| | | | Continuo us scale | | | 1.04 (0.93-1.14) | 5.06E-01 |
| | *Lautropia mirabilis* group | up | 3 (>0.70) | 190 | 81 | 1.79 (1.13-2.87) | **1. 44E-02** |
| | | | 2 (0.18-0.70) | 189 | 33 | 0.61 (0.35 -1.06) | 8.17E-02 |
| | | | 1 (<0.18) | 190 | 53 | ref | |
| | | | Continuo us scale | | | 1.14 (1.06-1.24) | **6.07E-04** |
| | *Neisseria meningitidi s* group | up | 3 (>0.64) | 190 | 92 | 1.89 (1.19-3.05) | **7.75E-03** |
| | | | 2 (0.07-0.64) | 189 | 30 | 0.66 (0.37-1.16) | 1.48E-01 |
| | | | 1 (<0.07) | 190 | 45 | ref | |
| | | | Continuo us scale | | | 1.13 (1.07-1.2) | **3.98E-05** |
| | *Streptococc* us *pneumoniae* group | up | 3 (>3.35) | 190 | 115 | 2.74 (1.68-4.56) | **7. 62E-05** |
| | | | 2 (1.75-3.35) | 189 | 22 | 0.62 (0.32-1.17) | 1.43E-01 |
| | | | 1 (<1.75) | 190 | 30 | ref | |
| | | | Continuo us scale | | | 1.11 (1.07-1.16) | **9.87E-08** |
| | *Streptococc* us *sanguinis* group | up | 3 (>1.79) | 190 | 101 | 2.14 (1.34-3.46) | **1. 55E-03** |
| | | | 2 (0.85-1.79) | 189 | 24 | 0.5 (0.27-0.91) | **2.67E-02** |
| | | | 1 (<0.85) | 190 | 42 | ref | |
| | | | Continuo us scale | | | 1.11 (1.04-1.18) | **1.60E-03** |
| | *Fusobacteri* um *periodontic* um group | down | 3 (>3.83) | 190 | 13 | ref | |
| | | | 2 (1.60-3.83) | 189 | 38 | 3.54 (1.75-7.59) | **6.91E-04** |
| | | | 1 (<1.60) | 190 | 116 | 9.26 (4.87-18.99) | **1.10E-10** |
| | | | Continuo us scale | | | 0.64 (0.56-0.72) | **2. 16E-11** |
| | *Haemophilus parainfluen* zae group | down | 3 (>13.01) | 190 | 35 | ref | |
| | | | 2 (7.80-13.01) | 189 | 26 | 0.54 (0.29-0.99) | **4.85E-02** |
| | | | 1 (<7.80) | 190 | 106 | 2.27 (1.4-3.77) | **1. 17E-03** |
| | | | Continuo us scale | | | 0.95 (0.92-0.98) | **4. 62E-04** |
| | *KV831974_s* group | down | 3 (>1.76) | 190 | 46 | ref | |
| | | | 2 (0.58-1.76) | 189 | 51 | 1.35 (0.8-2.3) | 2.64E-01 |
| | | | 1 (<0.58) | 190 | 70 | 1.98 (1.21-3.32) | **7.64E-03** |
| | | | Continuo us scale | | | 0.86 (0.77-0.95) | **3.78E-03** |
| | *Neisseria subflava* group | down | 3 (>17.85) | 190 | 36 | ref | |
| | | | 2 (7.87-17.85) | 189 | 39 | 0.83 (0.47-1.47) | 5.27E-01 |
| | | | 1 (<7.87) | 190 | 92 | 2 (1.22-3.31) | **6.24E-03** |
| | | | Continuo us scale | | | 0.97 (0.95-0.99) | **4.75E-03** |
| | *PAC001345_s* group | down | 3 (>2.29) | 190 | 16 | ref | |
| | | | 2 (0.65-2.29) | 189 | 33 | 2.21 (1.12-4.5) | **2.49E-02** |
| | | | 1 (<0.65) | 190 | 118 | 6.35 (3.51-12.19) | **4.63E-09** |
| | | | Continuo us scale | | | 0.74 (0.64-0.85) | **4.17E-05** |
| | *Porphyromon as pasteri* group | down | 3 (>3.80) | 190 | 21 | ref | |
| | | | 2 (0.92-3.80) | 189 | 44 | 1.99 (1.08-3.76) | **2.97E-02** |
| | | | 1 (<0.92) | 190 | 102 | 4.36 (2.5-7.93) | **5.19E-07** |
| | | | Continuo us scale | | | 0.85 (0.79-0.92) | **5.22E-05** |
| | *Prevotella histicola* | down | 3 (>1.36) | 190 | 34 | ref | |
| | | | 2 (0.15-1.36) | 189 | 37 | 1.1 (0.61-1.98) | 7.48E-01 |
| | | | 1 (<0.15) | 190 | 96 | 3.46 (2.1-5.87) | **2.07E-06** |
| | | | Continuo us scale | | | 0.9 (0.82-0.97) | **1.30E-02** |
| | *Prevotella melaninogen ica* | down | 3 (>7.30) | 190 | 17 | ref | |
| | | | 2 (3.13-7.30) | 189 | 33 | 2.2 (1.13-4.45) | 2.32E-02 |
| | | | 1 (<3.13) | 190 | 117 | 6.57 (3.66-12.49) | 1. 51E-09 |
| | | | Continuo us scale | | | 0.83 (0.78-0.88) | **4.39E-09** |
| | *Prevotella nanceiensis* group | down | 3 (>1.67) | 190 | 10 | ref | |
| | | | 2 (0.53-1.67) | 189 | 37 | 3.72 (1.77-8.48) | **9.18E-04** |
| | | | 1 (<0.53) | 190 | 120 | 10.01 (5.07-21.84) | **4.42E-10** |
| | | | Continuo us scale | | | 0.59 (0.47-0.73) | **2.39E-06** |
| | *Prevotella pallens* | down | 3 (>2.23) | 190 | 18 | ref | |
| | | | 2 (0.67-2.23) | 189 | 44 | 2.33 (1.25-4.49) | **9.19E-03** |
| | | | 1 (<0.67) | 190 | 105 | 4.87 (2.75-9.03) | **1. 55E-07** |
| | | | Continuo us scale | | | 0.68 (0.57-0.78) | **7. 92E-07** |
| | *Prevotella salivae* | down | 3 (>1.33) | 190 | 37 | ref | |
| | | | 2 (0.31-1.33) | 189 | 55 | 1.61 (0.95-2.74) | 7.67E-02 |
| | | | 1 (<0.31) | 190 | 75 | 2.07 (1.24-3.48) | **5.67E-03** |
| | | | Continuo us scale | | | 0.82 (0.69-0.96) | **1.59E-02** |
| | *Prevotella_* uc | down | 3 (>1.51) | 190 | 23 | ref | |
| | | | 2 (0.32-1.51) | 189 | 53 | 2.32 (1.31-4.24) | **4.90E-03** |
| | | | 1 (<0.32) | 190 | 91 | 3.77 (2.19-6.72) | **3.28E-06** |
| | | | Continuo us scale | | | 0.75 (0.63-0.86) | 2.55E-04 |
| | *Streptococc* us *salivarius* group | down | 3 (>1.06) | 190 | 44 | ref | |
| | | | 2 (0.30-1.06) | 189 | 43 | 1.15 (0.67-1.99) | 6.07E-01 |
| | | | 1 (<0.30) | 190 | 80 | 2.04 (1.26-3.38) | **4.50E-03** |
| | | | Continuo us scale | | | 0.95 (0.86-1.05) | 3.15E-01 |
| | *Veillonella dispar* | down | 3 (>11.02) | 190 | 30 | ref | |
| | | | 2 (3.46-11.02) | 189 | 69 | 2.21 (1.31-3.82) | **3.57E-03** |
| | | | 1 (<3.46) | 190 | 68 | 2.08 (1.23-3.61) | **7.54E-03** |
| | | | Continuo us scale | | | 0.96 (0.93-0.99) | **5.30E-03** |
| | *Veillonella parvula* group | down | 3 (>4.28) | 190 | 33 | ref | |
| | | | 2 (1.47-4.28) | 189 | 51 | 1. 57 (0.92-2.71) | 1.01E-01 |
| | | | 1 (<1.47) | 190 | 83 | 1.89 (1.13-3.2) | **1. 66E-02** |
| | | | Continuo us scale | | | 0.93 (0.87-0.99) | **2.12E-02** |

^{a} Tertiles for each metabolite were divided based on the distribution among control groups only. ^{b} Multiple conditional logistic regression (MCLR) was adjusted for sex, age, smoking, and drinking status. ^{c} P-value was calculated by Kruskal-Wallis using a false discovery rate (FDR) developed by Benjamini-Hochberg. OR: odds rate; 95% CI: confidence interval

At the species level, in the upregulated group, *Fusobacterium nucleatum, Lautropia mirabilis, Neisseria meningitidis, Streptococcus pneumoniae,* and *Streptococcus sanguinis* were significantly associated with increased risk of oral cancer, with higher risks identified in the highest tertile compared to the lowest tertile. In the downregulated group, *Fusobacterium periodonticum, Haemophilus parainfluenzae,* KV831974_s, *Neisseria subflava,* PAC001345_s, *Porphyromonas pasteri, Prevotella histicola, Prevotella melaninogenica, Prevotella nanceiensis, Prevotella pallens, Prevotella salivae, Prevotella_uc, Veillonella dispar,* and *Veillonella parvula* showed significant negative associations with higher risks identified in the lowest tertile compared to the highest tertile.

### (2) Functional imbalance and network analysis

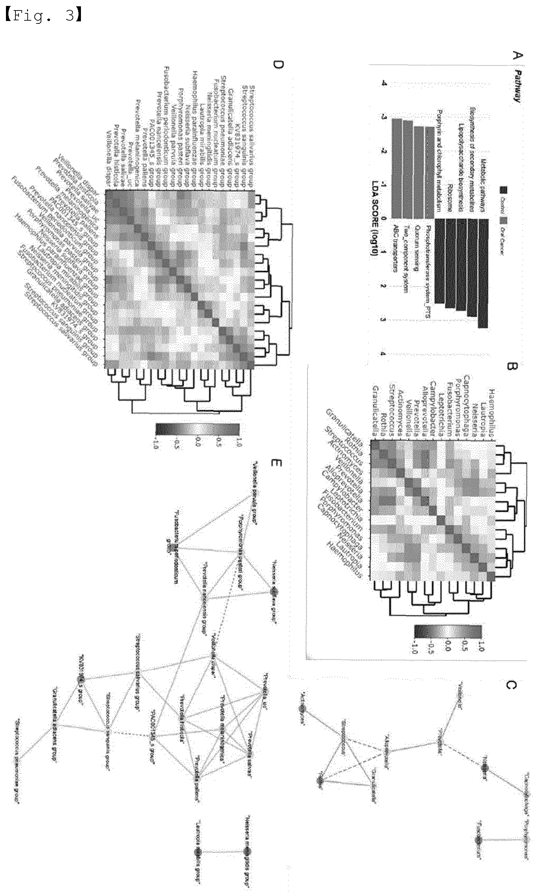

From all samples of 736 participants, a total of 446 KEGG pathways were identified using the Phylogenetic Investigation of Communities by Reconstruction of Unobserved States (PICRUSt) embedded reference database, and then LEfSe analysis was performed to study the functional changes in the oral cancer microbiota. As a result, 9 KEGG pathways were identified as significantly different between groups (FIG. 3A). 5 pathways (biosynthesis of lipopolysaccharides, porphyrin and chlorophyll metabolism, metabolic pathways, biosynthesis of secondary metabolites, and ribosomes) were enriched in the control group, while 4 pathways (quorum sensing, ABC transporter, Two_component system, and phosphotransferase system _PTS) were activated in patients with oral cancer.

As shown in FIG. 3B, there are two co-abundance groups at the genus level (group 1 includes *Actinomyces* and *Streptococcus,* while group 2 includes *Prevotella* and *Porphyromonas*). The stringent network, excluding weak correlations (|r| <0.4), also showed this association trend (FIG. 3C). At the species level, *Prevotella* spp. are strongly positively correlated with each other, while *Porphyromonas pasteri* is strongly correlated with species from other phyla (FIGS. 3D and 3E).

### Example 3. Biomarker identification based on genomic analysis

### 3.1. Model construction for biomarker identification

To identify biomarkers for oral cancer, a multistep feature screening was performed on genera and species with one or more sequences in 5% or more of all subjects.

Specifically, we performed a model that adopted a two stages machine-learning approach of Least Absolute Shrinkage and Selection Operator (LASSO) and random forest (RF), using the R packages "glmnet" and "random forest," respectively, to reduce features.

First, LASSO efficiently selected relevant taxa by adjusting lambda parameters to improve model accuracy and interpretability, and then RF was applied to a subset of important taxa selected by the LASSO algorithm to generate the final model. To obtain an unbiased estimate of model performance, a five-fold cross validation (CV) was used to prevent over-fitting bias. Within each iteration of the five-fold CV, the dataset was randomly divided into 5 parts and 4 parts were combined as a training set to generate a predictive model, while the remaining 1 part was used as a test set to validate model performance. In LASSO, the number of features was reduced by using a model with λ less than 1 standard error of the minimum. In RF, an optimistic number of trees were selected by training the model with sequences from 40 to 400 using a default threshold defined by the model. Taxa were then selected based on a mean decrease accuracy model.

### 3.2. Identification and validation of oral cancer diagnostic biomarkers

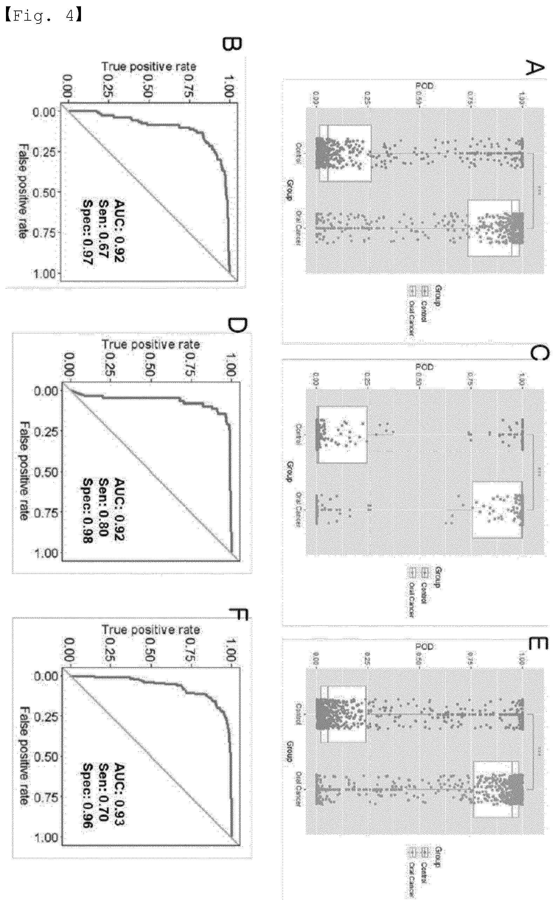

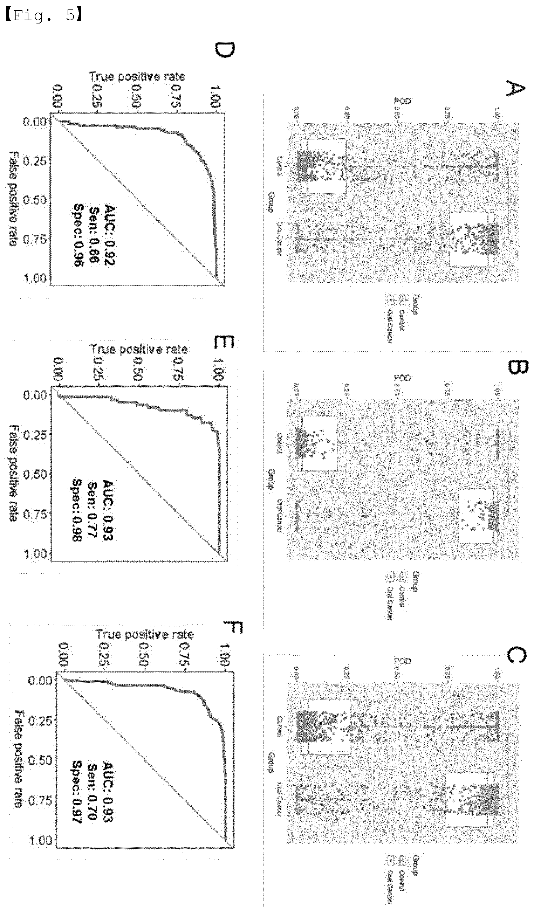

To identify genus- and species-based biomarkers for oral cancer, a machine learning model was used on the discovery set consisting of a two-step procedure of the least absolute shrinkage and selection operator (LASSO) feature selection, followed by random forest (RF). The same dataset for LEfSe analysis was input and used in this process, and ten-fold cross-validation (CV) was applied to both LASSO and RF. At the genus level, 18 genera were selected as the optimal genus panel using machine learning (Table 4). In the discovery set (106 patients; 381 controls), the probability of disease (POD) index was significantly higher in patients with cancer compared to healthy control groups (FIG. 4A), and this POD index achieved an area under the curve (AUC) value of 0.92 (sensitivity 0.67; specificity 0.97) (FIG. 4B). Similarly, in the validation set (61 patients; 188 controls), POD was also significantly higher in the patient group (FIG. 4C), which achieved an AUC value of 0.92 (sensitivity 0.80; specificity 0.98) (FIG. 4D). To further confirm the diagnostic potential of this panel of genus biomarkers, the POD index and AUC were also calculated for the entire dataset (167 patients; 569 controls) . The POD index was significantly higher in the oral cancer group with an AUC value of 0.93 (sensitivity 0.70; specificity 0.96) (FIGS. 4E and 4F). At the same time, AUC values were also calculated for single genera. Among the 18 genera, *Prevotella* and *Streptococcus,* in particular, exhibited a high AUC value of 0.7 or more, and may serve as potential single biomarkers of oral cancer (Table 4). At the species level, the 16 species were selected as the optimal set of biomarkers (Table 4). The POD index was significantly higher in the oral cancer group in all datasets (FIGS. 5A to 5C). The AUC values were 0.92 (sensitivity 0.66; specificity 0.96), 0.93 (sensitivity 0.77; specificity 0.98), and 0.93 (sensitivity 0.70; specificity 0.97) for the discovery, validation, and total sets, respectively (FIGS. 5D to 5F). Six of the 16 species (AF385567_s, *Fusobacterium periodonticum, Prevotella melaninogenica, Prevotella pallens, Streptococcus constellatus,* and *Streptococcus pneumoniae*) may also be potential single markers for oral cancer (AUC >= 0.7) (Table 4). It can be seen that the 5 genera (*Actinomyces, Capnocytophaga, Porphyromonas, Prevotella, Streptococcus)* and the 4 species (*Fusobacterium periodonticum* group, *Prevotella melaninogenica, Prevotella pallens, Streptococcus pneumoniae* group) were consistently repeated in all analyses.

**[Table 4]**

| Taxon level | Taxon name | Median abundance | | Fold change (FC) | | AUC | | |
|---|---|---|---|---|---|---|---|---|
| | | Patient s | Control group | Patie nts/C ontro l group | P-value adjuste d value^{a} | Disc over y set | Vali dati on set | Full set |
| Genus | *Abiotrophia* | 0.00 | 0.00 | 2.45 | 8.52E-07 | 0.61 | 0.64 | 0.62 |
| | *Actinomyces* | 1.18 | 0.82 | 1.43 | 1.16E-02 | 0.57 | 0.59 | 0.57 |
| | *Alloscardovi a* | 0.00 | 0.00 | NA | 6.76E-08 | 0.66 | 0.51 | 0.61 |
| | *Capnocytopha ga* | 1.26 | 0.73 | 1.72 | 8.52E-07 | 0.56 | 0.75 | 0.63 |
| | *Dialister* | 0.18 | 0.08 | 2.11 | 1.43E-08 | 0.61 | 0.70 | 0.65 |
| | *Howardella* | 0.00 | 0.00 | NA | 3.11E-23 | 0.66 | 0.76 | 0.69 |
| | *Leuconostoc* | 0.00 | 0.00 | NA | 1.23E-25 | 0.64 | 0.67 | 0.65 |
| | *Mannheimia* | 0.01 | 0.02 | 0.80 | 9.63E-01 | 0.48 | 0.53 | 0.50 |
| | *Mogibacteriu m* | 0.05 | 0.03 | 1.55 | 5.48E-06 | 0.59 | 0.66 | 0.61 |
| | *Olsenella* | 0.00 | 0.00 | NA | 5.55E-09 | 0.64 | 0.63 | 0.63 |
| | *Paraburkhold eria* | 0.00 | 0.00 | NA | 6.56E-25 | 0.58 | **0.87** | 0.69 |
| | *Parvimonas* | 0.25 | 0.07 | 3.49 | 6.46E-11 | 0.65 | **0.71** | 0.67 |
| | *Pasteurellac eae uc* | 0.08 | 0.17 | 0.49 | 1.96E-02 | 0.43 | 0.44 | 0.44 |
| | *Porphyromona s* | 1.77 | 3.55 | 0.50 | 8.52E-07 | 0.68 | 0.53 | 0.62 |
| | *Prevotella* | 9.92 | 16.95 | 0.59 | 3.22E-14 | 0.66 | **0.77** | **0.70** |
| | *Saccharimona s* | 0.05 | 0.10 | 0.48 | 1.26E-06 | **0.70** | 0.50 | 0.38 |
| | *Sphingomonas* | 0.00 | 0.00 | NA | 1.09E-22 | 0.61 | **0.84** | 0.69 |
| | *Streptococcu* s | 9.77 | 4.94 | 1.98 | 1.52E-17 | **0.70** | **0.76** | **0.72** |
| Species | *AF385567_s* | 0.00 | 0.00 | NA | 3.54E-23 | 0.66 | **0.76** | **0.70** |
| | *Actinomyces odontolyticu* s group | 0.57 | 0.49 | 1.15 | 6.17E-01 | 0.51 | 0.52 | 0.51 |
| | *Alloprevotel la tannerae* | 0.10 | 0.09 | 1.13 | 2.02E-01 | 0.57 | 0.46 | 0.54 |
| | *Capnocytopha ga sputigena* group | 0.20 | 0.07 | 2.69 | 5.52E-07 | 0.57 | **0.73** | 0.63 |
| | *Dialister pneumosintes* | 0.06 | 0.02 | 3.69 | 4.35E-11 | 0.63 | 0.75 | 0.67 |
| | *Fusobacteriu m periodonticu m* group | 0.82 | 2.68 | 0.31 | 4.93E-22 | 0.75 | 0.74 | 0.75 |
| | *Lactobacillu s iners* | 0.00 | 0.00 | NA | 9.14E-09 | 0.58 | 0.52 | 0.56 |
| | *Lactobacillu s sakei* group | 0.00 | 0.00 | NA | 4.89E-20 | 0.63 | 0.64 | 0.64 |
| | *Leuconostoc gelidum* group | 0.00 | 0.00 | NA | 7.38E-22 | 0.61 | 0.57 | 0.59 |
| | *Neisseria elongata* group | 0.18 | 0.18 | 1.04 | 7.53E-01 | 0.49 | 0.54 | 0.51 |
| | *Paraburkhold eria graminis* group | 0.00 | 0.00 | NA | 3.11E-31 | 0.57 | **0.86** | 0.68 |
| | *Prevotella baroniae* | 0.02 | 0.01 | 2.45 | 2.02E-02 | 0.57 | 0.56 | 0.56 |
| | *Prevotella melaninogeni ca* | 1.70 | 5.13 | 0.33 | 7.38E-22 | **0.75** | **0.77** | **0.74** |
| | *Prevotella pallens* | 0.29 | 1.37 | 0.21 | 4.70E-15 | 0.69 | **0.71** | **0.70** |
| | *Streptococcu s constellatus* group | 0.09 | 0.03 | 3.33 | 1.53E-16 | 0.68 | **0.77** | **0.71** |
| | *Streptococcu s pneumoniae* group | 5.07 | 2.40 | 2.11 | 3.49E-17 | 0.69 | **0.80** | **0.72** |

### Example 4. Confirmation of diagnostic effectiveness in early-stage oral cancer

Analysis was performed on the oral microbiome, which is considered as a potential single marker for oral cancer diagnosis, and two orthologs that showed significant differences in expression between patients with oral cancer and control groups, for early-stage oral cancer diagnosis. The data were analyzed after log2 normalization.

### 4.1. Oral microbiome

An analysis was performed on the oral microbiome, namely strains of the genus *Howardella,* the genus *Paraburkholderia,* the genus *Porphyromonas,* the genus *Capnocytophaga,* and the genus *Sphingomonas,* which are significant for the diagnosis of oral cancer, in the early diagnosis of oral cancer. The staging of oral cancer was divided into stages 1 to 4 according to the size (T) of the primary cancer, with stages 1 and 2 being early-stage oral cancer, and stages 3 and 4 being late-stage oral cancer.

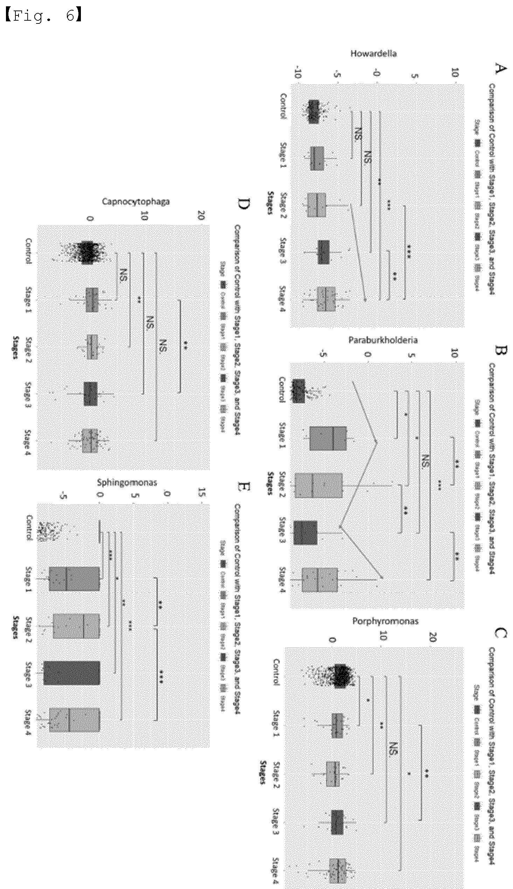

The results showed that strains of the genus *Howardella* were relatively higher in stage 4 compared to stage 2 (p < 0.001). However, there was no significant difference in expression between stage 1 and other stages (FIG. 6A).

For strains of the genus *Paraburkholderia,* a significant difference in expression was observed between the control groups and patients with oral cancer grouped according to stage, except for stage 3. In addition, the expression level of *Paraburkholderia* was relatively higher in stage 2 compared to stage 3 (p < 0.01), and a significant difference in expression was also observed between stage 1 and stage 2, and between stage 3 and stage 4, respectively (FIG. 6B).

For strains of the genus *Porphyromonas,* a significant difference in expression was observed between the control groups and patients with oral cancer grouped according to stage, except for stage 3. In addition, the expression levels were relatively higher in stage 1 compared to stage 3 (p < 0.01) (FIG. 6C).

For strains of the genus *Capnocytophaga,* a relatively high expression ratio was observed in stage 2 between the control groups and patients with oral cancer grouped according to stage compared to the control group (p < 0.01). It also showed relatively lower expression in stage 3 compared to stage 1 (p < 0.01) (FIG. 6D).

For strains of the genus *Sphingomonas,* a significant difference in expression was observed between the control groups and patients with oral cancer grouped according to stage. It also showed that the expression level of *Sphingomonas* was relatively lower in stage 4 compared to stage 2 (p < 0.001), which can support the effectiveness of *Sphingomonas* for the diagnosis of early-stage oral cancer (FIG. 6E).

Furthermore, to evaluate the performance of the five oral microbiomes mentioned above as biomarkers for early oral cancer diagnosis, receiver operating characteristics (ROC) curve analysis was performed.

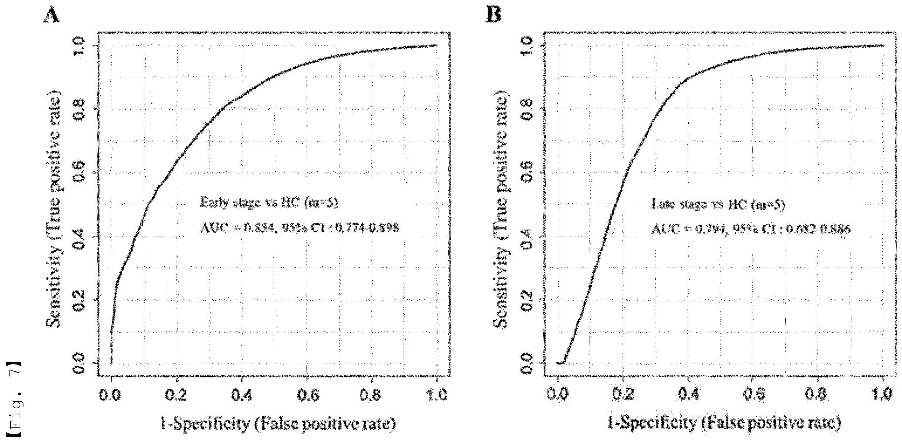

As a result, it was confirmed from FIG. 7 that the five oral microbiomes exhibited higher performance, particularly in the diagnosis of early-stage oral cancer, with an AUC of 0.834 for early-stage oral cancer compared to an AUC of 0.794 for late-stage oral cancer.

### 4.2. Orthology

An ortholog refers to a gene that has evolved through speciation while retaining its original function from a common ancestor, meaning that a gene with a specific function in any one species has the same function in another species.

To identify orthology-based biomarkers for early oral cancer diagnosis, we analyzed orthologs in early and late stages of oral cancer that exhibited significant differences between patients with oral cancer and healthy control groups. Specifically, a difference in expression was confirmed between the control groups and patients with oral cancer grouped into stages 1 to 4 according to the oral cancer stage.

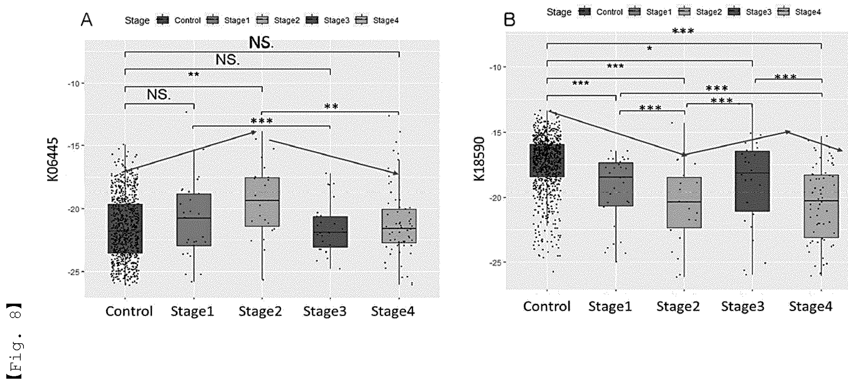

As a result, it was confirmed that among the two orthologs, acyl-CoA dehydrogenase exhibited higher expression levels in stages 1 and 2 compared to the control group. It was confirmed that dihydrofolate reductase exhibited significant differences in all stages 1 to 4, with notably lower expression levels in stages 1 and 2 compared to the control group. In addition, the level of dihydrofolate reductase was significantly lower in stage 2 compared to stage 3 (p < 0.001). Thus, (acyl-CoA dehydrogenase (K06445), dihydrofolate reductase (K18590)) were confirmed to have the potential as biomarkers that can distinguish between early-stage and late-stage oral cancer (FIG. 8).

From the above description, those skilled in the art to which the present disclosure pertains will understand that the present disclosure can be implemented in other specific forms without changing its technical idea or essential features. In this regard, it should be understood that the embodiments described above are exemplary in all respects and are not intended to be limiting. The scope of the present disclosure should be interpreted that all changes or modifications derived from the meaning and scope of patent claims to be described below rather than the detailed description above and their equivalent concepts are included within the scope of the present disclosure.

## Claims

1. A biomarker composition for diagnosing oral cancer, comprising at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae.*

2. The biomarker composition for diagnosing oral cancer of claim 1, further comprising at least one microorganism selected from the group consisting of the genus *Granulicatella,* the genus *Lautropia,* the genus *Alloprevotella,* the genus *Fusobacterium,* the genus *Haemophilus,* the genus *Veillonella, Fusobacterium nucleatum, Granulicatella adiacens, Haemophilus parainfluenzae,* KV831974_s, *Lautropia mirabilis, Neisseria meningitidis, Neisseria subflava,* PAC001345_s, *Porphyromonas pasteri, Prevotella histicola, Prevotella nanceiensis, Prevotella salivae, Prevotella_uc, Streptococcus salivarius, Streptococcus sanguinis, Veillonella dispar,* and *Veillonella parvula*

3. The biomarker composition for diagnosing oral cancer of claim 1, further comprising at least one microorganism selected from the group consisting of the genus *Dialister,* the genus *Mogibacterium,* the genus *Parvimonas,* the genus *Mannheimia,* the genus *Pasteurellaceae_uc,* the genus *Saccharimonas, Actinomyces odontolyticus, Alloprevotella tannerae, Capnocytophaga sputigena, Dialister pneumosintes, Neisseria elongate, Prevotella baroniae,* and *Streptococcus constellatus.*

4. The biomarker composition for diagnosing oral cancer of claim 1, wherein the biomarker composition comprises an agent for detecting the microorganism.

5. The biomarker composition for diagnosing oral cancer of claim 1, further comprising at least one microorganism selected from the group consisting of the genus *Howardella,* the genus *Paraburkholderia,* and the genus *Sphingomonas.*

6. The biomarker composition for diagnosing oral cancer of claim 5, wherein the biomarker composition comprises an agent for detecting at least one microorganism selected from the group consisting of the *genus Howardella,* the *genus Paraburkholderia,* the *genus Porphyromonas,* the *genus Capnocytophaga,* and the genus *Sphingomonas,*
wherein the oral cancer is early-stage oral cancer.

7. The biomarker composition for diagnosing oral cancer of claim 1, further comprising an agent for confirming an expression level of a gene encoding at least one of acyl-CoA dehydrogenase and dihydrofolate reductase,
wherein the oral cancer is early-stage oral cancer.

8. A kit for diagnosing oral cancer, comprising the biomarker composition for diagnosing oral cancer of claim 1.

9. The kit for diagnosing oral cancer of claim 8, wherein the kit diagnoses oral cancer based on the abundance of microorganisms contained in a biological sample.

10. A method for providing information for diagnosis of oral cancer, comprising:
(a) analyzing the abundance of at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae* in a biological sample; and
(b) comparing the abundance of the microorganism analyzed in step (a) with the abundance of the microorganism analyzed in a control sample.

11. The method for providing information for diagnosis of oral cancer of claim of 10, wherein the biological sample is saliva.

12. The method for providing information for diagnosis of oral cancer of claim of 10, wherein DNA is extracted from the biological sample to confirm the abundance of the microorganisms.

13. The method for providing information for diagnosis of oral cancer of claim of 10, further comprising: analyzing the abundance of at least one additional microorganism selected from the group consisting of the genus *Granulicatella,* the genus *Lautropia, Fusobacterium nucleatum, Granulicatella adiacens,* the genus *Alloprevotella,* the genus *Fusobacterium,* the genus *Haemophilus,* the genus *Veillonella, Haemophilus parainfluenzae,* KV831974_s, *Lautropia mirabilis, Neisseria meningitidis, Neisseria subflava,* PAC001345_s, *Porphyromonas pasteri, Prevotella histicola, Prevotella nanceiensis, Prevotella salivae, Prevotella_uc, Streptococcus salivarius, Streptococcus sanguinis, Veillonella dispar,* and *Veillonella parvula* in the sample, in step (a).

14. The method for providing information for diagnosis of oral cancer of claim of 10, further comprising: analyzing the abundance of at least one additional microorganism selected from the group consisting of the genus *Dialister,* the genus *Mogibacterium,* the genus *Parvimonas, Actinomyces odontolyticus, Alloprevotella tannerae, Capnocytophaga sputigena, Dialister pneumosintes, Neisseria elongate, Prevotella baroniae, Streptococcus constellatus,* the genus *Mannheimia,* the genus *Pasteurellaceae_uc,* and the genus *Saccharimonas* in the sample, in step (a).

15. The method for providing information for diagnosis of oral cancer of claim of 10, further comprising: analyzing the abundance of at least one additional microorganism selected from the group consisting of the genus *Howardella,* the genus *Paraburkholderia,* and the genus *Sphingomonas* in the sample, in step (a).

16. The method for providing information for diagnosis of oral cancer of claim of 15, wherein the analyzing of the abundance of at least one additional microorganism selected from the group consisting of the genus *Howardella,* the genus *Paraburkholderia,* the genus phyromonas, the genus pnocytophaga and the genus *Sphingomonas* in the sample is for diagnosing early-stage oral cancer.

17. The method for providing information for diagnosis of oral cancer of claim of 10, further comprising: confirming an expression level of a gene encoding at least one of an acyl-CoA dehydrogenase and a dihydrofolate reductase in step (a),
wherein the oral cancer is early-stage oral cancer.

18. A method for selecting a candidate drug for treating, ameliorating, or preventing oral cancer, comprising:
(a) analyzing the abundance of at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica , Prevotella pallens,* and *Streptococcus pneumoniae* in a biological sample obtained from a subject before treatment with a candidate drug for treating, ameliorating, or preventing oral cancer;
(b) analyzing the abundance of at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae* in a biological sample obtained from a subject after treatment with the candidate drug; and
(c) screening the candidate drug by comparing the abundance of the microorganism analyzed in step (a) with the abundance of the microorganism analyzed in step (b).

19. A method for predicting or monitoring oral cancer treatment response, comprising:
(a) analyzing the abundance of at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae* in a biological sample obtained from a patient with oral cancer;
(b) analyzing the abundance of at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae* in a biological sample obtained from a patient after oral cancer treatment; and
(c) predicting or monitoring a treatment response by comparing the abundance of the microorganism analyzed in step (a) with the abundance of the microorganism analyzed in step (b).

20. A probiotic composition for treating, ameliorating, or preventing oral cancer, comprising at least one microorganism selected from the group consisting of the genus *Porphyromonas,* the genus *Prevotella, Fusobacterium periodonticum, Prevotella melaninogenica, Prevotella pallens,* the genus *Alloprevotella,* the genus *Fusobacterium,* the genus *Haemophilus,* the genus *Veillonella, Haemophilus parainfluenzae, KV831974_s, Lautropia mirabilis, Neisseria meningitidis, Neisseria subflava,* PAC001345_s, *Porphyromonas pasteri, Prevotella histicola, Prevotella nanceiensis, Prevotella salivae, Prevotella_uc, Streptococcus salivarius, Veillonella dispar, Veillonella parvula,* the genus *Mannheimia,* the genus *Pasteurellaceae_uc,* and the genus *Saccharimonas.*

21. A method for diagnosing oral cancer, comprising:
(a) analyzing the abundance of at least one microorganism selected from the group consisting of the genus *Actinomyces,* the genus *Capnocytophaga,* the genus *Porphyromonas,* the genus *Prevotella,* the genus *Streptococcus, Fusobacterium periodonticum, Prevotella melaninogenica, Prevotella pallens,* and *Streptococcus pneumoniae* in a biological sample; and
(b) comparing the abundance of the microorganism analyzed in step (a) with the abundance of the microorganism in a control sample.

22. Use of the composition of any one of claims 1 to 7, for diagnosing oral cancer.

23. A method of treating, ameliorating, or preventing oral cancer, comprising administering to an individual the composition of claim 20.

24. Use of the composition of claim 20, for the manufacture of a medicament for treating, ameliorating, or preventing oral cancer.
